# EUROPEAN PATENT APPLICATION

(11) **EP 2 120 171 A2**
(43) Date of publication of application: **18.11.2009**
(21) Application number: 09006473.4
(22) Date of filing: 13.05.2009
(51) Int. Cl.: G06F 19/00

(54) **Methods, systems and a platform for managing medical data records**

(30) Priority: 14.05.2008 US 71708 P
(71) Applicant: Algotec Systems Ltd., 43107 Raanana (IL)
(72) Inventor: Benjamin, Menashe, 43555 RaAnana (IL); Bauer, Ori, 55525 Kiryat-Ono (IL); Velan, Noam, 45296 Hod-HaSharon (IL)
(74) Representative: Becker Kurig Straus

(57) **Abstract**

A method for providing an imaging study at a client terminal. The method comprises receiving a request for an imaging study from a client terminal connected to a first system of a plurality of medical imaging systems and identifying a destination of a device hosting the requested imaging study. The device is disparately connected to a second system of the plurality of medical imaging systems. The method further comprises acquiring the imaging study from the hosting device using the destination and forwarding the imaging study to the client terminal.

## Description

### RELATED APPLICATIONS

This application is related to U.S. Provisional Patent Application, Attorney Docket No. 43831, entitled "DISTRIBUTED INTEGRATED IMAGE DATA MANAGEMENT SYSTEM", filed on even date as this application, the disclosure of which is incorporated herein by reference.

### FIELD AND BACKGROUND OF THE INVENTION

The present invention, in some embodiments thereof, relates to a system and a method for managing medical data and, more particularly, but not exclusively, to a system and a method for managing access and/or storage of medical data.

Systems and devices for visualizing the inside of living organisms are among the most important medical developments in the last thirty years. Systems like computerized tomography (CT) scanners and magnetic resonance imaging (MRI) scanners allow clinicians to examine internal organs or areas of the body that require a thorough examination. In use, the visualizing scanner outputs a 3D imaging study, such as a sequence of computerized cross-sectional images of a certain body organ, which is then interpreted by specialized radiologists.

Commonly, a patient is referred for a visual scan by a general practitioner or an expert practitioner. The 3D imaging study is forwarded to and diagnosed by a general radiologist who is responsible for the analysis and diagnosis of the imaging study. Radiologists are trained to deal with all kinds of imaging studies, such as those of the brain, abdomen, spine, chest, pelvis and joints. The imaging studies and the diagnosis thereof are sent back to the referring practitioner. It should be noted that there are private diagnostic imaging centers (DICs) that supply radiology imaging services to whoever is interested.

In most hospitals and radiology centers, the 3D imaging studies are transferred to a picture archiving communication system (PACS) before being accessed by the radiologists. The PACS is installed on one or more of computers, which are dedicated for storing, retrieving, distributing and presenting the stored 3D imaging studies. The 3D imaging studies are stored in an independent format. The most common format for image storage is digital imaging and communications in medicine (DICOM). Usually, the PACS stores imaging studies on DICOM servers that provide central storage and access to the images. The DICOM servers are connected to the PACS network and allow radiologists and other healthcare staff which are connected thereto via client terminals which are connected to the PACS network in Clinics, hospitals and/or home settings to access the imaging studies.

PACS are often implemented on network systems, such as local area networks (LANs). Such a PACS includes a server system for controlling the transfer of imaging study data from DICOM servers to multiple client terminals of the LAN. Since imaging study files are typically large data files, it is not uncommon for simultaneous data requests from multiple client systems to heavily burden the existing bandwidth of the network's data link.

### SUMMARY OF THE INVENTION

According to some embodiments of the invention, there is provided a method for providing an imaging study at a client terminal. The method comprises receiving a request for an imaging study from a client terminal connected to a first system of a plurality of medical imaging systems, identifying a destination of a device hosting the imaging study, the hosting device being disparately connected to a second system of the plurality of medical imaging systems, acquiring the imaging study from the hosting device using the destination, and forwarding the imaging study to the client terminal.

Optionally, each the medical imaging system is a picture archiving and communication system (PACS).

Optionally, the hosting device is a member selected from a group consisting of: a radiology information system (RIS), an electronic medical record (EMR) system, a digital imaging and communications in medicine (DICOM) server, a computerized tomography (CT) modality, a magnetic resonance imaging (MRI) modality, and a positron emission tomography (PET)-CT modality.

Optionally, the imaging study is of a patient, the request comprises a request for medical information related to the patient, the acquiring comprising acquiring the medical information from the hosting device using the destination, and the forwarding comprising forwarding the medical information to the client terminal.

More optionally, the medical information is a record selected from a group consisting of a hospital information system (HIS) and a radiology information system (RIS).

Optionally, each the imaging study comprises a plurality of layers, the acquiring comprising sequentially acquiring the plurality of layers, and the forwarding comprising sequentially forwarding the plurality of layers.

More optionally, the forwarding allows displaying at least a portion of the imaging study according to the forwarded layers during the acquiring.

Optionally, the method further comprises associating a medical report with the imaging study.

Optionally, the acquiring comprises receiving a stream of the imaging study from the hosting device.

According to some embodiments of the invention, there is provided a platform for managing a plurality of imaging studies. The platform comprises a first integration device connected to a first medical imaging system having a plurality of first client terminals, and a second integration device connected to a second medical imaging system having a storage device configured for storing a plurality of imaging studies. The first integration device is configured for establishing a connection with the second integration device via a network, each the first client terminal being configured for acquiring at least one of the imaging studies via the connection.

Optionally, the platform further comprises a central node connected to the network and configured for documenting the addresses of the plurality of imaging studies, the first integration device being configured for using the central node for establishing the connection.

Optionally, the central node is configured for identifying an address of the storage device in a global list documenting addresses of the plurality of imaging studies, the acquiring being performed according to the address.

Optionally, the central node comprises a right management module configured for identifying a usage right in relation to the first integration device and allowing the establishing according to the identified usage right.

More optionally, the platform further comprises a plurality of integration devices each disparately connected to a medical imaging system having a plurality of client terminals, the first and second integration devices being part of the plurality of integration devices.

More optionally, at least one of the plurality of integration devices is disparately connected to a medical information system storing a plurality of medical records, each the first integration device is configured for acquiring at least one of the plurality of medical records via the connection.

More optionally, the plurality of imaging studies are associated with a plurality of patients, each the integration device is configured for receiving a request for medical information pertaining to a first of the plurality of patients from a first of the plurality of client terminals and for acquiring a group of the plurality of imaging studies accordingly, each member of the group being associated with the first patient.

According to some embodiments of the invention, there is provided a method for managing medical imaging studies. The method comprises receiving, at a storage device, a request for an imaging study from a client terminal, transmitting the imaging study to the requesting client terminal to allow a displaying thereof by the requesting client terminal, recording the transmission, receiving a medical report pertaining to the imaging study from the requesting client terminal, and associating the medical report with the imaging study.

Optionally, the method further comprises receiving an additional request for the imaging study from an additional client terminal and notifying the additional client terminal about the transmission.

Optionally, the medical report is stored in a member selected from a group consisting of: an electronic mail, a short message service (SMS), and an instant messaging (IM).

According to some embodiments of the invention, there is provided a method for acquiring medical information pertaining to a selected patient. The method comprises receiving a request for medical information pertaining to a selected patient at a client terminal, identifying a match between the request and a list of a plurality of medical data records of a plurality of disparate medical imaging systems, using the match for acquiring a group of the plurality of medical data records, each member of the group pertaining to the selected patient, and displaying the group at the client terminal.

Optionally, the group comprises members acquired from at least two of the disparate medical imaging systems.

Optionally, the plurality of medical data records are selected from a group consisting of a radiology information system (RIS) object, an electronic medical record (EMR) object, a digital imaging and communications in medicine (DICOM) object, a computerized tomography (CT) image, a magnetic resonance imaging (MRI) image, and a positron emission tomography (PET)-CT image.

Optionally, the method further comprises allowing a user to edit at least one member of the group.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data. Optionally, a network connection is provided as well. A display and/or a user input device such as a keyboard or mouse are optionally provided as well.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1 is a schematic illustration of a system for managing medical data records which are stored in multiple local medical imaging systems, according to some embodiments of the present invention;
Fig. 2 is a schematic illustration of an integration device that is connected to a local medical imaging system, according to some embodiments of the present invention;
Fig. 3 is a flowchart of method for allowing a user to use a client terminal of a first local medical imaging system to access medical data records which are stored in a second local medical imaging system, according to some embodiments of the present invention;
Fig. 4A is an exemplary data flow chart that depicts a process made according to the method depicted in Fig. 3, according to some embodiments of the present invention;
Fig. 4B is another exemplary data flow chart that depicts a process that is similar to the described in relation to Fig. 4A, according to some embodiments of the present invention;
Fig. 5 is a schematic illustration of the platform that is depicted in Fig. 1 with a central network node, according to some embodiments of the present invention;
Fig. 6 is a schematic illustration of the platform that is depicted in Fig. 5 where the central network node is hosted in one of the local medical imaging systems, according to some embodiments of the present invention; and
Fig. 7 is a data flow chart of a method for streaming layered medical data records, according to some embodiments of the present invention.

### DESCRIPTION OF SPECIFIC EMBODIMENTS OF THE INVENTION

The present invention, in some embodiments thereof, relates to a system and a method for managing medical data and, more particularly, but not exclusively, to a system and a method for managing access and/or storage of medical data.

According to some embodiments of the present invention, there is provided a platform for managing medical data which distributed and optionally created, edited and/or deleted in multiple local medical imaging systems, such as local PACS. The platform and the method provide a centralized solution with a synchronized workflow throughout a multi site enterprise.

Optionally, such a platform has the ability to synchronize multiple existing PACSs and other radiology information systems. Such a platform enables clinicians, such as radiologists, to log onto a user interface that allows them to access medical data records, such as imaging studies and reports from disparate PACSs and other radiology information systems.

According to some embodiments of the present invention, there is provided a method for providing an imaging study at a client terminal. The method is based on receiving a request for a medical data record, such as an imaging study, at a client terminal that is connected to a first local medical imaging system, such as a picture archiving and communication system (PACS) network, identifying a destination of the requested medical data record at a storage device which is connected to a second local medical imaging system, and using the received destination for forwarding the request to the storage device via a computer network. These actions allow the receiving of the requested medical data record image from the storage device via the computer network. In such an embodiment, a user, such as a clinician, for example a radiologist, or a computer added diagnosis (CAD) system which are using a client terminal that is connected to one local medical imaging system to edit medical data records which are located in another local medical imaging system.

According to some embodiments of the present invention, the medical data records are layered. In such an embodiment, the client terminal may display one or more layers of a requested medical record during the transferring of additional layers thereof. In such an embodiment, the platform enables relatively prompt access to imaging studies from any location as a result of the ability to send layers of a certain imaging study in a sequential manner, even over low bandwidth networks.

According to some embodiments of the present invention there is provided a platform and a method for managing the distribution of medical data records and/or copies thereof among a plurality of local medical imaging systems.
The aforementioned embodiments allow clinicians, such as radiologists, to initiate diagnosis and/or reporting sessions using various client terminals which may be dispersed in large regional or national geographies. The architecture of the platform allows managing the storage of imaging studies and other medical data records in a plurality of medical imaging systems.

According to some embodiments of the present invention there is provided a method for acquiring medical information pertaining to a selected patient from a plurality of disparate medical imaging systems. The method is based on receiving a request for medical information pertaining to the selected patient at a certain client terminal and identifying a match between the request and a global list or a number of local lists of a plurality of medical data records of the disparate medical imaging systems. The identification of the match allows the acquiring a group of the plurality of medical data records, where each member of the group is related to the selected patient. The group is then displayed at the client terminal to a clinician. Such an embodiment allows a clinician to base his diagnosis on medical data records from a plurality of remote and disparate medical information resources.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

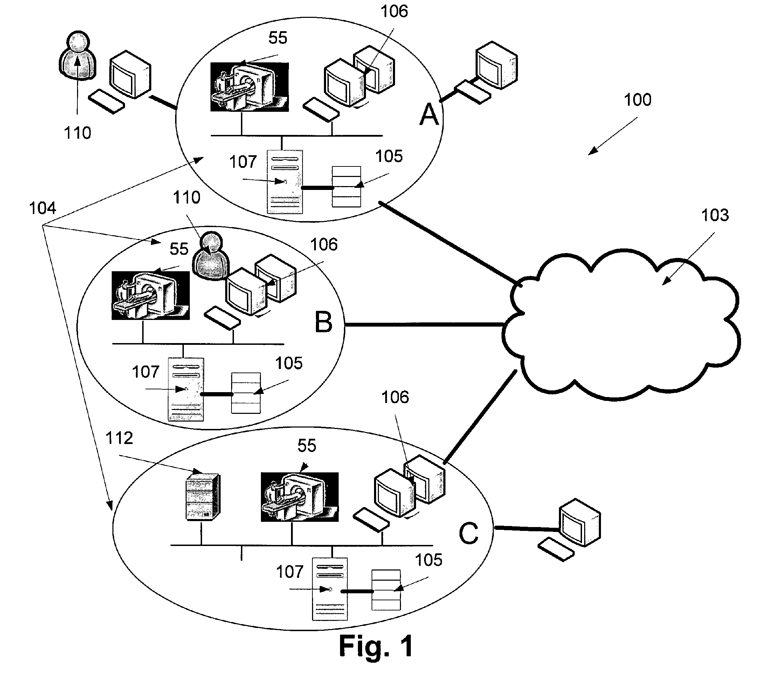

Reference is now made to Fig. 1, which is a schematic illustration of a platform 100 for managing medical data records which are stored and optionally created, edited and/or deleted, in multiple local medical imaging systems 104, according to some embodiments of the present invention. The platform 100 is a distributed database that manages a database of medical data records in a plurality of storage devices which may be dispersed over a network of interconnected computers.

The platform 100 includes a plurality of integration devices 107 which are connected to one another via a network 103, such as the Internet. The integration device 107 interfaces between client terminals 106 and medical information resources of disparate local medical imaging systems 104. The integration device 107 is optionally defined as described in a co-filed patent application by the same inventors, entitled "Distributed Integrated Image Data Management System" filed on even date as this application, which is incorporated herein by reference. As disclosed in the co-filed patent application, the integration device 107 may be installed in an independent computing unit, such as a server, which is connected to one or more of the related local medical imaging system 104. As further disclosed in the co-filed patent application, the integration device 107 may be integrated into the local medical imaging system 104. In such an embodiment, the local medical imaging system 104 includes the software and/or hardware components of the integration device 107.

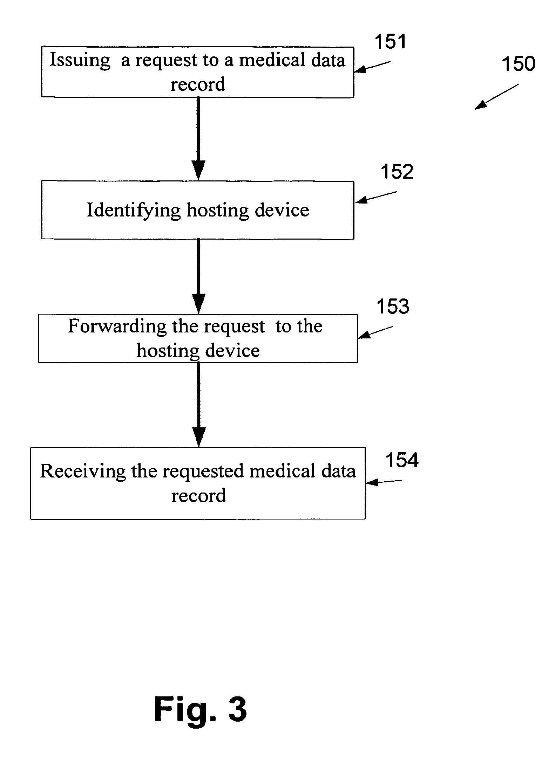

The integration devices 107 of the platform 100 are designed to communicate with one another, optionally as described below in relation to Fig. 3, Fig. 4A, and Fig. 7.

The communication between each one of the integration devices 107 and the client terminals and/or storage devices of the respective local medical imaging system 104 is performed according to known communication protocols and standards, such as the DICOM standard, which is incorporated herein by reference.

In some embodiments of the present invention one or more of the local medical imaging systems 104 are PACS networks. In such embodiments, each local medical imaging system consists of a central server, such as a DICOM server, that stores a plurality of imaging studies, such as outcomes of medical imaging, for example imaging studies, and connected to one or more client terminals 106, such as personal computers, laptops, thin clients, smartphones, and/or personal digital assistants (PDAs), optionally via a LAN and/or a wide area network (WAN).

Each local medical imaging system 104, which is optionally an independent PACS, may include web-based interfaces to utilize the Internet or any other computer network, as a device of communication, for example via a virtual private network (VPN) or a secure sockets layer (SSL) connection. In such an embodiment, each local medical imaging system 104 may include a number of client terminals 106 which are located in a remote location. For example, a PACS of a hospital may allow the hospital radiologists to access DICOM objects which are stored in a local DICOM server from a client terminal 106, such as a personal computer, that is located in his office, his home, and/or in another hospital.

Client terminals 106 may use local peripherals for receiving, optionally by scanning, imaging studies into one of the storage units in the platform 100, optionally as further described below and/or for printing and/or displaying imaging studies from the platform 100 and interactive display of digital images. Optionally, the client terminals 106 offer means of manipulating the images, for example means for cropping, rotating, zooming, brightening, and contrasting. Each one of the local medical imaging systems 104 may be managed by a different entity, such as a hospital, a clinic, a group of hospitals, and/or a group of clinics.

The platform 100 allows a user, which is connected to a certain client terminal 106 of one of the local medical imaging systems 104, to access medical data, which is stored in a storage device 112 and/or a client terminal 106 that is associated with another of the local medical imaging systems 104. Such a storage device may be a server, such as a DICOM server, a database, and/or a client terminal 106 with local memory, such as a personal computer, a PDA and the like. For example, a clinician that uses a client terminal that is connected to local medical imaging system A, as shown at 110, may access, edit, update, add, and/or delete medical data that is stored on any storage device of the local medical imaging system, such as a server 112 or a client terminal 106, which is connected to another local medical imaging system, such as B or C.

As described above, the integration device 107 may be integrated into the local medical imaging system 104. Such an integration device 107 provides more control to the users of the system 100. Such integration allows the deleting of medical imaging studies or separate medical images thereof, splitting medical imaging studies or separate medical images thereof, and/or merging medical imaging studies or separate medical images thereof. Such integration further allows adding proprietary information, such as sticky notes, bookmarks of screen, treatment instructions, and/or teaching files to the medical imaging studies or to separate medical images thereof.

Optionally, one or more of the local medical imaging systems 104 are connected to a hospital information system (HIS), variously also called clinical information system (CIS), which is designed to manage the administrative, financial and/or clinical aspects of an entity, such as a hospital, that manages the related local medical imaging system 104. Optionally, one or more of the local medical imaging systems 104 is connected to a radiology information system (RIS). The RIS stores, manipulates and/or distributes patient radiological data and imagery that is related to patient associated with the respective local medical imaging system 104. As used herein, a medical data record means an imaging study, a RIS record, a HIS record, and/or any other record that includes medical information which is related to a certain clinician.

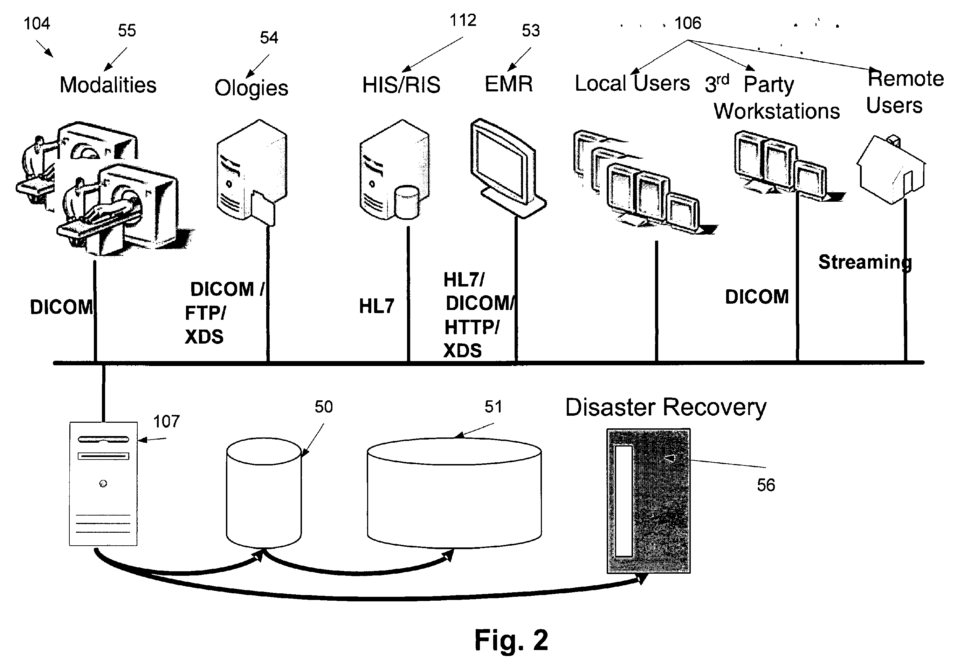

Reference is now also made to Fig. 2, which is a schematic illustration of an integration device 107 that is connected to a certain local medical imaging system 104, according to some embodiments of the present invention. As described above, each one of the integration devices 107 is designed to transfer local medical data records to other integration devices 107 and/or to receive similar medical data records therefrom. The integration device 107 is connected and/or associated with local client terminals 106, such as personal computers at remote client terminals, third party client terminals 106, or local terminals which are directly connected thereto. The integration device 107 may also be connected to local storage devices, such as local imaging study repositories, HIS, and/or RIS, as shown at 112. Optionally, the integration device 107 is connected electronic medical record (EMR) system 53 and/or any other Health Information Technology (HIT) which keep track of medical information, such as the practice management system which supports the electronic medical record. Optionally, the integration device 107 is connected to any database or a repository 54 that includes medical information. As used herein, medical information means, *inter alia,* information that is related to patients, such as laboratory results, therapeutic procedure records, clinical evaluations, age, gender, medical condition, ID, genetic information, patient medical record, data indicating of metabolism, blood pressure, patient history, sensitivities, allergies, different population records, treatment methods and the outcome thereof, epidemiologic classification, patient history, such as treatment history and any combination thereof.

Optionally, the integration device 107 is connected to radiology equipment 55 of the respective local medical imaging system 104. As used herein radiology equipment 55 means computerized tomography (CT), a positron emission tomography (PET)-CT, and/or magnetic resonance imager (MRI) modalities, and/or any other imaging systems which are designed to capture imaging studies, and optionally to feed them, for example using a film digitizer, directly to a storage unit. As used herein, an imaging study means a three dimensional (3D) imaging study, a four dimensional (4D) imaging study, a spatial image, a sequence of CT scan images, a sequence of MRI scan images, a sequence of PET-CT scan images, an imaging study with additional information layers, and a DICOM object.

The integration device 107 allows a clinician that uses a client terminal 106 of a first local medical imaging system to use medical data that is hosted in a RIS, an imaging studies repository, a HIS, and/or any medical data record that is stored in a client terminal 106 and/or a storage device of a second local medical imaging system. It should be noted that as each one of the local medical imaging systems has local storage devices, clinicians may use local client terminals 106 for accessing locally stored medical data.

As described above, each one of the local medical imaging systems 104 includes storage devices that host medical data records, such as imaging studies, RIS records, HIS records, and/or any other records of medical information. Optionally, the medical data records are prepared for low-bandwidth lines, optionally as further described below. Optionally, the preparation includes compressing medical data records such as imaging studies. Optionally, the preparation includes flexibly accommodate the displacement of packets of medical data records such as imaging studies by using a layered, or multi-resolution, representation. Packetizing each layer separately allows grained control of the transmission process. Optionally, the layering includes splitting a medical record, such as an imaging study into a coarse image base layer and one or more enhancement layers containing added details.

Optionally, the integration device 107 manages one or more repositories 50, 51, and 56. Optionally, each one of the integration devices 107 manages a repository 50, optionally a memory that allows relatively fast access to data, such as a flash memory, for storing a local dataset 105 that includes references to medical data records which are stored its local medical imaging system 104 and in other local medical imaging systems 104 which are connected to the platform 100. Optionally, the integration device 107 manages a dataset that includes references to all the medical data records, which are accessible via the local medical imaging system 104 which is connected thereto. The dataset may be referred to herein as a local grid 105, which may be referred to as a global list.

Optionally, each medical data record and/or reference may be associated with identification tag that is related to the patient to which it relates. The identification tag allows consolidating different medical data records of the same patient, creating a cluster of patient related medical data records which may be referred to herein as a patient cluster.

Each reference to a medical data record in the local grid 105 contains spotting data for identifying the physical location of the related medical data record. Optionally, the spotting data includes one or more of the following:
1. A master storage address - an internet protocol (IP) address, a uniform resource locator (URL), and/or any other address of the hosting storage device and/or of the integration device 107 of the local medical imaging system that hosts the requested medical data record. The master storage address may include a hosting device port, which is the port of the hosting storage device and/or of the integration device 107 of the local medical imaging system that hosts the requested medical data record.
2. A DICOM application entity title.
3. A path identifier - as described above, a clinician may use a client terminal 106 in one local medical imaging system for accessing medical records which are hosted in other local medical imaging systems. Optionally, the path identifier includes one or more addresses, such as hop addresses, of the integration device 107 of the local medical imaging system 104 that hosts the related medical data record. The path identifier may include the port of the integration device 107 and/or of any storage device that is used for storing the related medical data records.
   The path identifier may include a set of instructions that defines a path to the storage device that hosts the related medical record and/or the related integration device 107.
4. Security status - an indication whether the medical data is transferred over a secured connection, for example using secure sockets layer (SSL), or unsecured.
5. Connection downloading and/or uploading speed.

Optionally, one or more of the integration devices 107 manages a storage repository 51, such as a hard drive, for storing medical data records, for example as further described below.

Optionally, the integration devices 107 include a backup mechanism 56 for backing up the local grid 105 and/or the records which are stored in the storage repository 51. Optionally, the backup mechanism 56 assimilates backup solutions, such as a disaster recovery plan (DRP) sometimes referred to as a business continuity plan (BCP) or business process contingency plan (BPCP), which assure that should a disaster happens at one of the integration devices 107 or one of its repositories, the medical data records and/or the global grid, which are hosted in the related integration devices 107, will continue to be available via other integration devices 107 which are placed in another location.

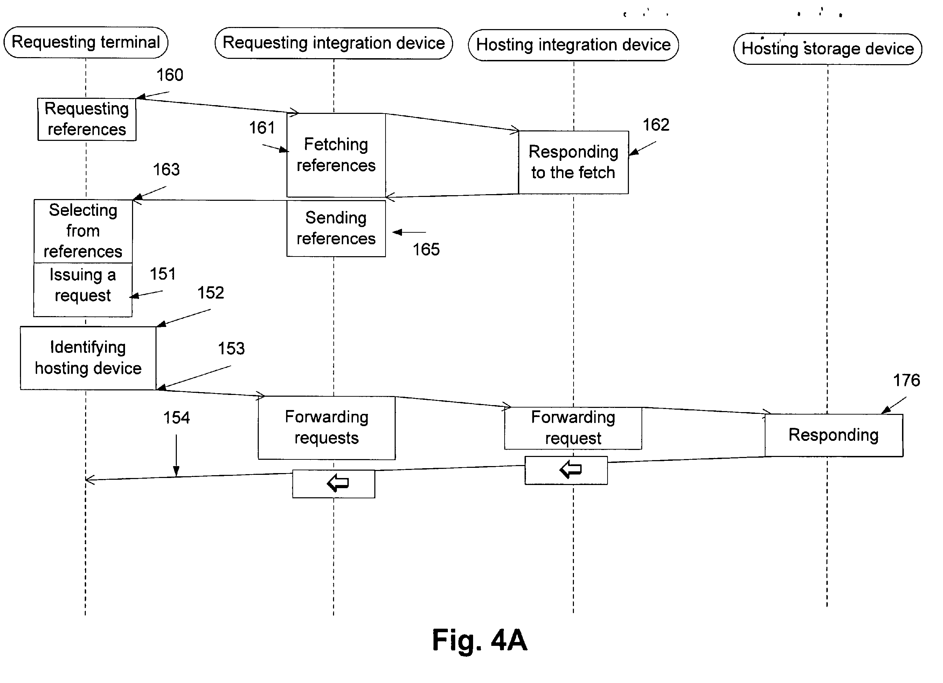

Reference is now made to Fig. 3, which is a flowchart 150 of method for allowing a clinician to use a client terminal 106 of a first local medical imaging system to access imaging studies and/or other medical data records which are stored in a second local medical imaging system, according to some embodiments of the present invention. Reference is also made to Fig. 4A, which is an exemplary data flow chart 170 that depicts a process made according to the method 150 which is depicted in Fig. 3, according to some embodiments of the present invention.

First, as shown at 151, a request for an imaging study is issued, optionally according to instructions of a user 110 at a client terminal 106 that is connected to the first local medical imaging system, such as A, B, and C. The client terminal 106 allows a user, such as a clinician 110 to select a patient cluster and/or a certain medical data record that is stored in any of the local medical imaging systems 104 which are connected to the platform 100, optionally according to her access rights.

Optionally, the client terminal 106 includes a user module that allows the clinician to make such a selection from the respective local grid 105. Such a user module is optionally designed to fetch the local grid 105 from the respective integration device 107. Optionally, the client terminal 106 includes a graphical user interface (GUI) that allows the clinician 110 to select a patient cluster and/or a certain medical data from the respective local grid 105.

In some embodiments of the present invention, , as shown at 160, the client terminal 106 requests references to medical data records from integration devices 107 which are connected to a related local medical imaging system 104. In such an embodiment, the respective integration device 107 may send a request to some or all of the other integration devices 107 and to receive in response some or all of the references of their local grids 105, optionally as shown at 161 and 162. In such a manner, the respective integration device 107 may be able to provide references to all the medical data records which may be managed by the platform 100. Optionally, the client terminal 106 includes a GUI that allows the clinician 110 to select a patient cluster and/or a certain medical data from references fetched from respective local grids 105, for example as shown at 163.

Optionally, each one of some or all of the received references includes spotting data, optionally as described above. The spotting data allows the requesting client terminal to locally identify the address of a hosting device that stores the requested medical data record, as shown at 152. Now, as shown at 153, the requesting client terminal 106 forwards requests to the identified destinations, optionally via its integration device 107 that establish a connection with the integration device 107 of the integration device 107 at the local medical imaging system 104 to which the respective storage device is connected. The identified destination is used for forwarding requests to the storage device that hosts the requested medical data records.

Optionally, the reference is associated with a number of hosting devices that store copies of the same medical data record. In such an embodiment, a hosting device may be selected by weighing a number of factors which a related thereto, such as the download bandwidth, the upload bandwidth, an time stamp that reflects the last time it was updated, the security status of the connection therewith and the like.

The requests are forwarded via the network 103, in a connection that is established between the integration devices 107, or via other communication networks. In some embodiments of the present invention, each destination includes the IP address to and/or the path of a hosting storage device of the local medical imaging system 104 that is associated with the storage device that hosts one or more of the requested medical data records. As shown at 154, the hosting storage device responses to the request. The response includes the requested medical data record. The response may or may not be forwarded via the integration device 107 of its local medical imaging system. The response may or may not be forwarded via the integration device 107 of the local medical imaging system of the requesting client terminal 106. Such an embodiment allows the client terminal to acquire medical data records from disparate medical imaging systems.

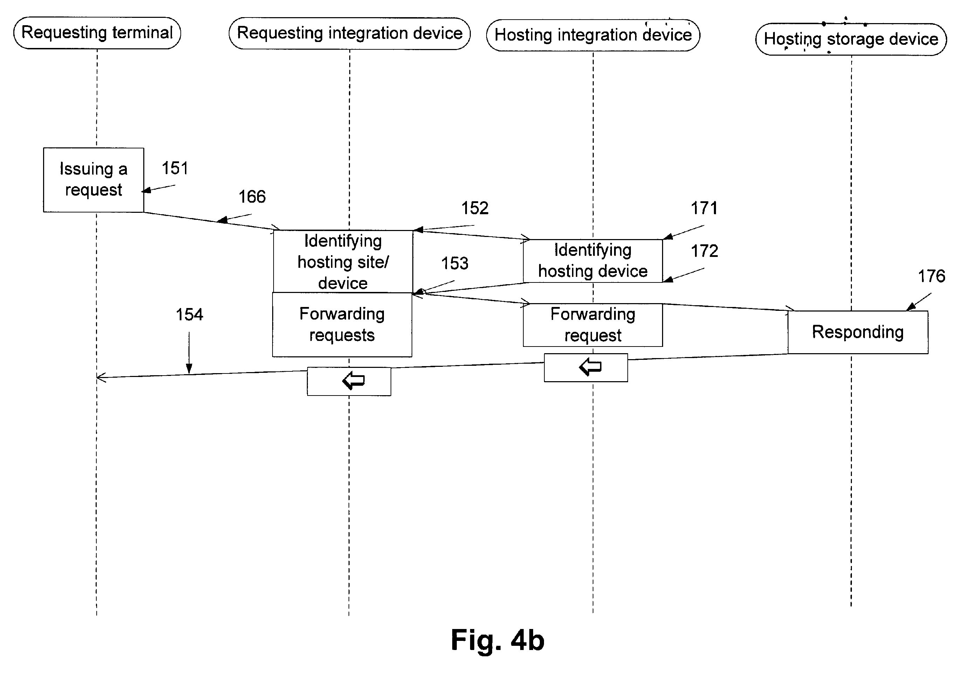

Reference is now made to Fig. 4B, which is an exemplary data flow chart that depicts a process that is similar to the process that is depicted in the data flow chart of Fig. 4A, according to some embodiments of the present invention. Fig. 4B depicts a process in which the address or the path of the hosting storage device is identified is identified by the integration device of the local site 104 of the requesting terminal 106. Optionally, the references are not fetched in advance and/or not fetched with sufficient spotting data for allowing the client terminal 106 to locally identify the hosting device.

In such an embodiment, the integration device 107 receives the issued request, as shown at 166, and searches references to the requested medical data records in its local grid 105. If references to the requested medical data records are found, the integration device 107 provides them to the requesting client terminal 106. In some embodiments of the present invention, if the spotting data in the local grid 105 includes an IP address that is stored in the local medical imaging system, the integration device 107 routes it accordingly. Else, as shown at 171, the integration device 107 forwards the request to one or more other integration devices 107. Each one of the other integration devices 107 searches references to the requested medical data records in its local grid. If the references are found, the integration device 107 provides them to the requesting integration device 107, as shown at 172. Else, the integration device 107 further forwards the request to one or more additional integration devices 107, optionally with the address of the requesting integration device 107 as the address of the requestor. This process may recur for a predefined number of iterations and/or until the requested references are found. When the requested references are found, they are transmitted to the requesting integration device 107, as shown at 172. Optionally, each one of the references includes a destination, such as a path and/or an IP address, of a hosting device. The destination may be a local destination of a local client terminal 106 or a local server, such as a DICOM server and/or a remote destination of a client terminal 106 or a local server which are connected to another local medical imaging system 104.

Now, as shown at 153, the requesting integration device 107 forwards requests to the identified destinations, optionally via the integration devices 107 of their local medical imaging systems 104. The identified destinations are used for forwarding requests to the storage devices that host the requested medical data records. The requests are forwarded via the network 103 or via other communication networks. In some embodiments of the present invention, each destination includes the IP address to and/or the path of a hosting storage device of the local medical imaging system 104 that is associated with the storage device that hosts one or more of the requested medical data records. As shown at 154, and described above the hosting storage device responses to the request.

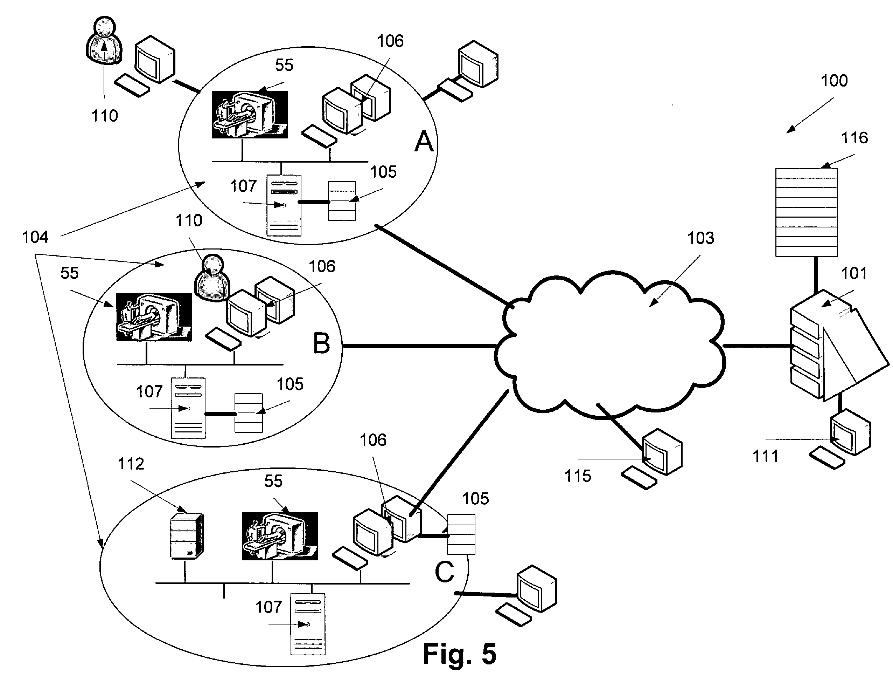

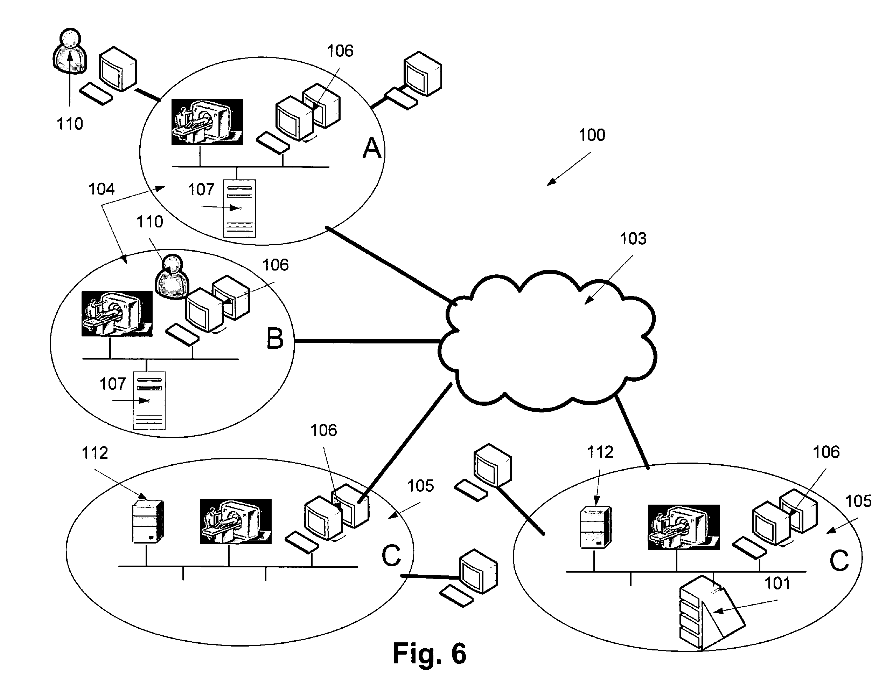

Reference is now made to Fig. 5, which is a schematic illustration of the platform 100 that is depicted in Fig. 1 with a central network node 101, according to some embodiments of the present invention. The central network node 101, which may be a central server and referred to herein as a central node 101, is connected to the network 103 and may be used for managing the access, and optionally the usage rights, of the medical data records which are hosted in the local medical imaging systems 104. Optionally, as shown at Fig. 6, the central network node 101 is hosted in one of the local medical imaging systems 104 and therefore may be directly connected to the client terminals 106 thereof. The central network node 101 is designed to communicate with the integration devices 107.

In such an embodiment, a clinician may use any client terminal 106 of any of the local medical imaging systems 104 and/or a client terminal 106 that is directly connected to the central node 101, as shown at 111 or to the network 103, as shown at 115, to access a medical data record that is stored in any of the local medical imaging systems 104.

Optionally, the central node 101 manages a repository for storing a dataset 116 that includes references to medical data records which are stored and/or accessible via one of the local medical imaging systems 104. The dataset may be referred to herein as a global grid 116.

As described above, the platform 100 uses integration devices 107 for allowing a clinician to use a client terminal 106 of a first local medical imaging system 104 to access a storage device, such as another client terminal 106 or a server, of a second local medical imaging system 104. Optionally, if the IP address of the hosting storage device and/or of the respective integration device 107 is not known, the path identifier includes the address of and/or a path to the central node 101.

As described above, the central node 101 hosts the global grid 116 that includes references that allows access to all the medical data records which are hosted in the platform 100. Optionally, the default path identifier is the IP address of the central node 101. If the path identifier directs the integration device 107 to the central node 101, the central node 101 searches for the IP address and/or for the path to the requested medical data record and updates the path identifier of the request and/or of the spotting data of the respective reference in the local grid 105. In such a manner, the integration device 107 of the requesting client terminal 106 is able to access the hosting local medical imaging system without using the central node 101 as a mediator and/or an interface.

As described above, the central node 101 is connected to the local medical imaging systems 104, either directly or via the integration devices 107. Optionally, such a communication allows a client terminal 106 of one of the local medical imaging systems 104 to acquire the global grid 116, or a portion thereof, and to display it to a local user. Optionally, the client terminal 106 is designed to display a list of patient clusters and/or a list of patient identifications which is based on the global grid 116. Such a list may allow the local user to request medical data records which are related to a patient selected from the global list.

In some embodiments of the present invention, the platform 100 allows the clinician 110 that is connected to a certain local medical imaging system 104 to add, delete, edit, and/or update medical data records which are stored in other local medical imaging systems 104. As used herein, editing means, *inter alia*, merging and splitting imaging studies which are related to a certain organ of a certain patient, adding sticky notes to a certain imaging study or record, and/or marking and/or tagging a structure and/or an element in an imaging study. It should be noted that some of the editing abilities may be determined according to the type of the local medical imaging system 104 that hosts the medical data records, for example as described above in relation to the editing abilities of a medical imaging system 104 that integrates the integration device 107.

For clarity, the marking and/or tagging may be performed by a clinician, such as a radiologist, and/or by a CAD system that is designed to analyze an imaging study and to mark suspicious structures and/or any anomaly therein.

As described above, the platform 100 is a distributed system in which medical data records are stored in a plurality of independent medical imaging system 104. In order to assure that the requesting client terminal 106 receives the medical data record it requests, a concurrency control that ensures that database transactions are performed concurrently without any concurrency violating the data integrity of medical data records is applied.

As described above, the platform 100 provides a clinician in a certain local medical imaging system access to medical data records which are hosted in other local medical imaging systems. Optionally, the access is provided by allowing the requesting client terminal 106 to download the requested medical data records, or a portion thereof, and to locally edit and/or update them. Optionally, the requesting creates a local copy of the requested medical data record that allows clinicians to edit and/or to reaccess them without the latency of accessing and/or editing remote records. In order to avoid redundant and/or anachronistic copies of the same medical data record, the central node 101 documents the copies of the medical data records. As described above, the local grid 105 includes references to local medical data records and to medical data records which are stored in storage devices and/or client terminal 106 which are associated with remote local medical imaging systems, and may be referred to herein as remote medical data records. Each reference to a medical data record associated with a copy/master field that indicates whether the reference is to a copy or to a master, a pointer to the master copy, and optionally with pointers to all the copies of the related medical data record. Each pointer is optionally associated with a timestamp that indicates the creation time of the local copy and/or with a list of changes made in the local copy. Optionally, when a local copy is acquired by a client terminal 106 of a certain local medical imaging system, the integration device 107 of the medical imaging system 104 of the requesting client terminal 106 updates the local grid 105 with a reference to the local copy. Optionally, the integration device 107 of the medical imaging system 104 of the hosting client terminal 106 updates the copy/master field and/or the one or more of the pointers to related references. In such an embodiment, when the local copy or the master of the related medical data record is requested by another client terminal 106, the client terminal 106 and/or the integration device 107 may retrieve the most up-to-date version of the related medical data record, Optionally, after the local copy is edited and/or updated, the editing and/or the updating are forwarded to the storage device of the master copy that adjust it accordingly. Optionally, as long as the editing and/or the updating are not forwarded, the master copy is locked to changes.

Optionally, the platform 100 allows the clinician 110 that uses a client terminal 106 connected to the certain local medical imaging system 104 to prepare a report that is related to one or the medical data records, for example to imaging studies which have been stored in other local medical imaging systems 104, and to store and associate the prepared report with the medical data records. In such an embodiment, the report is prepared at one of the client terminals 106 and forwarded to the respective integration device 107. The respective integration device 107 receives the report and sends it, optionally in a text format, to the integration device 107 at the medical imaging system 104 that hosts the related medical data record. The integration device 107 adds the report to a local storage device, optionally to the local RIS. Optionally the RIS is updated according to HL7 protocol, which the specification is incorporated herein by reference. It should be noted that the clinician may sent the report by an electronic mail, a short message service (SMS), an instant messaging (IM), and/or any other message in a digital format.

Optionally, the global grid 116 and/or the local grid 105 are backed up. Optionally, the platform 100 assimilates backup solutions, such as a DRP, for restoring the global grid 116 and/or the local grid 105, including regaining access thereto.

Optionally, the platform 100 in includes one or more right management modules that manage the access to the medical data records according to usage rights of subscribers. The one or more right management modules may be hosted in the central node 101 and/or in the integration devices 107. Each usage may include right for copying, deleting, recording, fixating, viewing, changing, and editing. In such a manner, the platform operator and/or the clinicians that add new medical data records may manage usage rights of different subscribers in relation to different medical data records and control the usage with the medical data records. In some embodiments of the invention, the platform operator may use the usage rights to differentiate between system users, for example to distinguish between subscribed and non-subscribed users or between different levels of subscribed users. Optionally, the platform 100 provides three different types of usage rights to the user:
- A local reading right - a usage right that allows a user to view medical data that is locally stored in storage devices which are associated with the respective local medical imaging system 104.
- A combined right - a usage right that includes the aforementioned local reading right and additional access and/or usage rights which are related to medical data that is related to the medical data that is stored in the respective local medical imaging system 104. For example, such a combined right may allow access to history information which is related to the locally stored medical data and/or to records which are related to the patient that is associated with the locally stored medical data.
- A global list right - a usage right that includes the aforementioned local reading right and additional rights which are determined according to a right management module which is optionally hosted by the central node 101. In such an embodiment, the right management module limits the usage rights a certain user according to a global list that includes usage rules which are related thereto. Such usage rules may define the usage rights which are associated with the user, for example whether she can access medical data of local medical imaging systems to which she is not directly connected and what are the usage right she has with respect to each local medical imaging system.

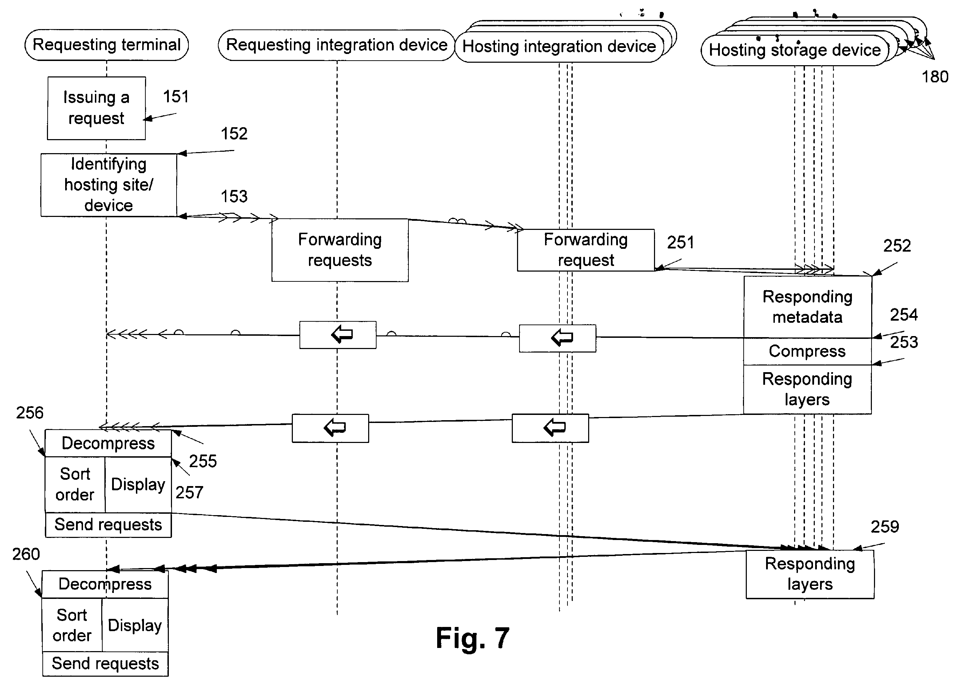

Reference is now made to Fig. 7, which is a data flow chart of a method for streaming layered medical data records, according to some embodiments of the present invention. Blocks and arrows 151, 152, and 153are as described in relation to Fig. 4A, however Fig. 7 further depicts actions which are related to layered medical data records which are acquired from a plurality of storage device which may be scattered in a plurality of local medical imaging systems 181, 182, 183, and 184. In some embodiments of the present invention, some or all of the medical data records are layered, optionally as outlined above.

Medical data records, such as imaging studies, may have a substantial size. The streaming and/or forwarding of such a substantial size require substantially large bandwidth and/or computational complexity. Furthermore, as described above, the client terminals 106 of the local medical systems which are connected to the platform 100 may be used for requesting for a plurality of medical data records, for example by requesting a patient cluster that includes a number medical data records which are related to a common patient, optionally from a number of local medical imaging systems. Such a request may require even more substantial bandwidth. As the platform 100 is designed to forward and/or to stream medical data records via computer networks, the clinician may have to wait for relatively long period before she receives a complete version of the requested medical records.

In order to avoid such a delay, the forwarded medical data records are layered. In such an embodiment, the storage devices that host the requested medical data records are designed for responding to a request by sending layers of the requested medical data. As shown at 251, after a request arrives to the storage device, the metadata of the requested medical data record, which may be referred to herein as a bogus header, is sent back to the requesting client terminal 106, optionally as shown at 252. In such an embodiment, as shown at 253, the storage units may send the base layer together or substantially together with the bogus header.

In some embodiments of the present invention, the requested medical data records are stored in a compress state. In such embodiments, the medical data records are decompressed before base layer is sent. Optionally, each storage unit compresses the layers of the requested medical data record before the transmission thereof, optionally as shown at 254. Optionally, the medical data record is compressed and has to be decompressed before it is layered. If the layer was compressed before the transmission there, the requesting terminal may decompress the layer of the requested medical data record upon receiving thereof, optionally as shown at 255.

Now, as shown at 256, the requesting client terminal 106 may calculate a current quality value for one or more of the requested medical data records according to the number of received layers. The current quality value may be defined between 0 and the number of layers that may be requested from a specific storage device in a single request and may be referred to herein as a requested quality value. Optionally, the requested quality value is a derivative of the type of the medical data record. Optionally, if the requested medical data record is layered, the requested quality value is graded. In such a manner, different values are given to the base layer, the second layer, the third layer, and the like. Request for additional layers, which are optionally issued as described below, are based on the layers which already have been received. For example, if the medical data record is a CT medical study and the requested medical data record is layered, the initial request is for medical data record with a quality value that is determined to reflect a lossy compression of 1:14 and the second request is for medical data record with a quality of medical image with a loosely compressed ratio of 1:7 and the final medical image with a quality of a lossless compression. It should be noted that the layers are accumulated at the requesting client terminal to achieve the desired quality.

Optionally, as long as the current quality value of a certain medical data record does not exceed a requested quality value which has been allocated thereto, the streaming thereof from the related storage device continues and new requests and responses thereto are forwarded between the integration devices, optionally as described below in relation to numerals 259 and 260. Optionally, a number of layers may be requested in each request. Optionally, the number of layers is determined according to the quality of the connection with the respective storage device.

As described above, an imaging study record may include an imaging study and the current quality value reflects the resolution of the received imaging study. The requested quality values of all the requested imaging studies allow the calculation of the total number of quality layers that have been requested in the request and/or the total number of quality layers which have been received in response to the request. Optionally, a maximum request capacity is defined per request. In such an embodiment, a request may be limited according to the maximum request capacity. Optionally, the session, during which a client terminal 106 requests and receives and the medical data records, is terminated if the total number of received quality layers exceeds the maximum request capacity.

In some embodiment of the present invention, a dynamic sorted list of medical data records is created according to the order of importance of the medical data records. Optionally, as outlined above and described below, each client terminal 106 hosts a UI that allows her to select medical data records and/or patient clusters to download. The graphical user interface (GUI) of the UI may include place holders and/or any other display options that allow her to focus on a selected medical data record. Optionally, the dynamic sorted list is sorted according to focus that is determined in the GUI.

The requested layers may be sorted according to their relative importance quality values, optionally during the receiving process. The sorting is optionally performed while the bogus headers and the layers are received and continuously throughout the loading session. Optionally, each requested medical data record that a bogus header thereof has been received is sorted according to one or more of the following characteristics:
the size of the layers of the medical data record - the smaller is the size of each layer, the higher is the position thereof in the sorted order;
whether the bogus header and/or the first layer of the medical data record is displayed, as described above, or not; and
whether the bogus header represents an image that is part of a sequence of images representing a 3D object or not.

In parallel to the sorting, as shown at 257, the requesting client terminal 106 may present the received base layers on a screen and/or any other display device that is associated with the requesting client terminal 106. In addition, when additional sequential layers are received, the display is updated. In such a manner, the clinician does not wait until all the requested medical data is received and can start diagnose requested medical data records, such as imaging studies, during the downloading and/or streaming process.

Now, as shown at 258, after the requested media records have been sorted, the client terminal 106 creates a plurality of request, each to the storage device that hosts the requested medical data record. Each request is optionally attached with respective spotting data. The requests are sent to the appropriate storage devices, optionally as described above in relation to numerals 151-153, 171, 172, and 173.

Optionally, each request is updated according to the layers which already have been received and according to the requested quality value thereof. Such a request may include the number of layers which have not been received and the related spotting data. In such a manner, the responses to the request may require less bandwidth. As shown at 259, the storage devices 180 respond to the requests. As shown at 260, new requests may be sent. This process recurs as long as more layers are needed to present the required medical data records in a predefined resolution. The requests are updated according to the received information and optionally according to actions of the clinician. For example, the requested quality value of a requested medical data record is updated according the focus that is determined by the clinician. Such an update affects the sorted order and respectively the order in which the requests are created and/or sent. Optionally, this process recurs as long as the aforementioned total number of layers has not exceeded. Optionally, medical data records with current quality values that exceed their requested quality values are removed from the list or tagged as having finished or arrived at completion.

Optionally, the client terminal 106 hosts a user module that includes a searching module that is designed to receive from the user indicia that defines a patient cluster and/or one or more medical data records. In such an embodiment, the searching module is designed to search the requested patient cluster and/or one or more medical data records in the local grid and/or to forward the indicia to the respective searching modules in other integration devices 107. In such an embodiment, the searching may be performed in a similar manner to the identification which is described above in relation to block 152.

Reference is now made, one again, to Fig. 5. As described above, the platform 100 allows a clinician, such as a radiologist and/or any other clinician to use a client terminal 106 that is associated with one local medical imaging system, to have access to medical data records which are located in other local medical imaging systems and optionally to edit, update and/or delete these medical data records. The radiologist may use her client terminal 106 to download these medical data records to his client terminal 106 and to locally analyze and/or update them. In such a manner, the client terminal 106 allows the radiologist to select and/or download medical data records regardless to the physical location thereof.

Optionally, the UI, which is executed by the client terminal 106, allows the clinician to download medical data records which are related to a certain patient from a number of different local medical imaging systems 104. For example, the UI may acquire medical data records, via the network 103, from a first local medical imaging system 104 that hosts new medical imaging data such as a DICOM object, a second local medical imaging system 104 that hosts RIS records, and a third local medical imaging system 104 that hosts old medical imaging data, such as archived DICOM objects.

Optionally, after a patient cluster has been selected and before all the related medical data records have been received, the client terminal 106 displays empty place holders for imaging studies according to a selected hanging protocol that defines the format and the presentation of the imaging study. Optionally, the hanging protocol consisted of layout information to display the imaging studies on the screen by the type of projection method. As described above, the requested medical data records may include medical information, such as patient history. The selected patient history, which may be acquired from the same local medical imaging system 104 as the imaging studies or from another local medical imaging system 104, is also displayed to the clinician.

As described above, updates, deletions, and additions which are made by requesting client terminal 106, are forwarded to the storage device that stores a master copy of the deleted, edited and/or updated medical data record, optionally according to the master storage address thereof. The storage device revises the master copy and/or address accordingly.

In some embodiments of the present invention, medical data records may be transferred from one local medical imaging system 104 to another by a distribution managing module, which is optionally hosted in the central node 101. Optionally, the global grid 116 of the central node 101 documents the requests which are made for each reference. The distribution managing module analyzes the distributed medical records and estimates the medical imaging system 104 in which it may be required. In such a manner, the distribution of the medical data records may be managed in a manner that reduces the number of data retrievals with high geographical communication latency that is needed to allow a number of remotely located client terminals 106 to receive access to the plurality of medical data records. Optionally, the distribution managing module transfers a certain medical data record to the local medical imaging system 104 from which it has been requested the most, optionally in a predefined period. Optionally, the transferred medical data records are transferred to the integration device 107 of the local medical imaging system 104 which has been estimated as the local medical imaging system 104 from which most of the requests with be sent. In such an embodiment, the integration device 107 may locally store the transferred medical data records and update its local grid 105 accordingly. Optionally, only local copies of the medical data records are transferred. Optionally, only a copy of the metadata, the base layer, and/or few additional layers of the medical data records are transferred to the local medical imaging system 104 which has been estimated as the local medical imaging system 104 from which most of the requests with be sent.

Optionally, the distribution managing module is designed to receive initiatory requests from the client terminals 106, 115 and/or 111. Each initiatory request includes a reference of a medical data record which may be requested at a certain local medical imaging system in a certain time. The distribution managing module optionally transfers the referred medical data record or a copy thereof to the certain local medical imaging system, before the certain time. In such an embodiment, a radiologist may make sure that a certain medical data record is stored in a certain local medical imaging system at a certain day and/or an hour. Optionally, the distribution managing module is designed to manage the storage of a certain medical data record, patient cluster, and/or any portion of the medical data record or the patient cluster. It should be noted that the transfer may be determined according to the characteristics of the content of the medical data record, such as the structure and the size thereof. In such an embodiment, different policies may be employed for different elements of the medical data records.

It is expected that during the life of a patent maturing from this application many relevant system and methods will be developed and the scope of the term an imaging study, a medical data record, a client terminal, a storage device and/or a network are intended to include all such new technologies *α priori.*

As used herein the term "about" refers to ± 10 %.

The terms "comprises", "comprising", "includes", "including", "having" and their conjugates mean "including but not limited to".

The term "consisting of means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention. To the extent that section headings are used, they should not be construed as necessarily limiting.

## Claims

1. A method for providing an imaging study at a client terminal, said method comprising:
receiving a request for an imaging study from a client terminal connected to a first system of a plurality of medical imaging systems;
identifying a destination of a device hosting said imaging study, said hosting device being disparately connected to a second system of said plurality of medical imaging systems;
acquiring said imaging study from said hosting device using said destination;
and
forwarding said imaging study to said client terminal.

2. The method of claim 1, wherein said imaging study is of a patient, said request comprises a request for medical information related to said patient, said acquiring comprising acquiring said medical information from said hosting device using said destination, and said forwarding comprising forwarding said medical information to said client terminal.

3. A method according to claims 1 or 2, wherein each said imaging study comprises a plurality of layers, said acquiring comprising sequentially acquiring said plurality of layers, and said forwarding comprising sequentially forwarding said plurality of layers.

4. A method according to any of claims 1-3, wherein said forwarding allows displaying at least a portion of said imaging study according to said forwarded layers during said acquiring.

5. A method according to any of claims 1-4, further comprising associating a medical report with said imaging study.

6. A platform for managing a plurality of imaging studies, comprising:
a first integration device connected to a first medical imaging system having a plurality of first client terminals; and
a second integration device connected to a second medical imaging system having a storage device configured for storing a plurality of imaging studies,
wherein said first integration device is configured for establishing a connection with said second integration device via a network, each said first client terminal being configured for acquiring at least one of said plurality of imaging studies via said connection.

7. The platform of claim 6, further comprising a central node connected to said network and configured for documenting the addresses of said plurality of imaging studies, said first integration device being configured for using said central node for establishing said connection.

8. The platform of claim 7, wherein said central node is configured for identifying an address of said storage device in a global list documenting addresses of said plurality of imaging studies, said acquiring being performed according to said address.

9. A platform according to any of claims 7-8, wherein said central node comprises a right management module configured for identifying a usage right in relation to said first integration device and allowing said establishing according to said identified usage right.

10. A platform according to any of claims 7-9, further comprising a plurality of integration devices each disparately connected to a medical imaging system having a plurality of client terminals, said first and second integration devices being part of said plurality of integration devices.

11. A method for managing medical imaging studies, comprising:
receiving, at a storage device, a request for an imaging study from a client terminal;
transmitting said imaging study to said requesting client terminal to allow a displaying thereof by said requesting client terminal;
recording said transmission;
receiving a medical report pertaining to said imaging study from said requesting client terminal; and
associating said medical report with said imaging study.

12. The method of claim 11, further comprising receiving an additional request for said imaging study from an additional client terminal and notifying said additional client terminal about said transmission.

13. A method according to claim 11 or 12, wherein said medical report is stored in a member selected from a group consisting of: an electronic mail, a short message service (SMS), and an instant messaging (IM).

14. A method for acquiring medical information pertaining to a selected patient, comprising:
receiving a request for medical information pertaining to a selected patient at a client terminal;
identifying a match between said request and a list of a plurality of medical data records of a plurality of disparate medical imaging systems;
using said match for acquiring a group of said plurality of medical data records, each member of said group pertaining to said selected patient; and
displaying said group at said client terminal.

15. The method of claim 14, wherein said group comprises members acquired from at least two of said disparate medical imaging systems.
